# EUROPÄISCHE PATENTSCHRIFT

(11) **EP 2 813 541 B1**
(45) Veröffentlichungstag und Bekanntmachung des Hinweises auf die Patenterteilung: **19.08.2020**
(21) Anmeldenummer: 14171591.2
(22) Anmeldetag: 06.06.2014
(51) Int. Cl.: C08J 9/24, A61L 27/56

(54) **FORMTEILE AUS PMMA-PULVER ALS EINFACHE DOSIERHILFE BEI DER HERSTELLUNG VON DENTALPROTHESEN**
Moulded parts made of PMMA powder as simple dosing aid in the manufacture of dental prostheses
Pièces de formage à base de poudre de PMMA en tant qu'aide au dosage facile pour la fabrication de prothèses dentaires

(30) Priorität: 10.06.2013 DE 102013106018
(43) Veröffentlichungstag der Anmeldung: 17.12.2014
(73) Patentinhaber: Kulzer GmbH, 63450 Hanau (DE)
(72) Erfinder: Dr. Ruppert, Klaus, 63477 Maintal (DE); Kerscher, Kevin, 61267 Neu-Anspach (DE); Dekert, Stephan, 61273 Wehrheim/Obernhain (DE)
(74) Vertreter: Bendele, Tanja

(56) Entgegenhaltungen:
- CH-A- 208 847
- DE-T2- 69 914 994
- US-A- 4 536 158

## Beschreibung

Die Erfindung betrifft dreidimensionale formstabile Formkörper von polymeren Partikeln, deren Partikel an den Kontaktpunkten der Partikel untereinander zumindest teilweise miteinander fest verbunden sind. Die vorkonfektionierten Formkörper aus polymeren, pulverförmigen Partikeln insbesondere aus PMMA können Acrylat oder MMA in die poröse dreidimensionale Struktur aufnehmen und bilden einen pastösen oder cremeartigen, gieß-, preß- oder injizierbaren Teig, der nach Formung mit üblichen Methoden gehärtet werden kann. Gleichfalls Gegenstand der Erfindung sind die Verwendung und ein Kit zur Herstellung des pastösen Teigs. Zur Anwendung kommen die vorkonfektionierten Formkörper in Verfahren zur Herstellung von Prothesen, wie Dentalprothesen, Knochenzementen, von Einbettmassen, insbesondere von porösen Substraten, als Einbettmasse in der Metallographie.

Medizintechnische Prothesen, wie Dentalprothesen oder Knochenprothesen, werden durch Vermischen eines Pulvers, das im Wesentlichen aus PMMA (Poly(methyl-2-methylpropenoat) besteht, und einer Flüssigkeit, die hauptsächlich aus MMA besteht, hergestellt. Trotz genauer Vorgaben der Hersteller in der Gebrauchsanleitung über das Mischungsverhältnis von Pulver und Flüssigkeit hält sich die Mehrzahl der Nutzer nicht genau an die Dosieranleitung, sondern dosiert "nach Gefühl" über die Viskosität der gebildeten entstehenden Mischung.

Ein wesentliches Problem bilden die sich zwangsläufig ergebenden Schwankungen in den Werkstoffeigenschaften, im Farbeindruck und im Schrumpfungsverhalten der hergestellten Prothesen. Ein weiteres Problem ist der Gehalt an Restmonomeren, der sich auch in der nichtstöchiometrischen Umsetzung ergibt, wenn der Nutzer sich nicht an die Dosieranleitung hält. Unmittelbar mit dem Restmonomergehalt verknüpft ist eine höhere gesundheitliche Belastung der Patienten durch diese Monomere. Zudem können durch den erwähnten Schrumpf Paßungenauigkeiten der zahntechnischen Arbeit auftreten.

CH 208847 offenbart die Herstellung medizinischer Prothesen, indem ein zerkleinertes, festes Polymerisat umfassend Acrylsäurebutylester mit einem Gemisch flüssiger Monomere umfassend Acrylsäurebutylester und Methacrylsäuremethylester vermengt und polymerisiert wird. Reine Acrylsäuremethylester sind toxikologisch bedenklich, DE69914994 offenbart die Herstellung eines porösen, polymeren Materials, das auf der kovalenten Vernetzung von Organolgelpartikeln mit Hydroxyl-Gruppen mit Diphenylmethandiisocyanat beruht.

US4536158 offenbart das Verkleben eines PMMA-Pulvers zu einem porösen Formkörper durch Beschichtung mit einem hydrophilen Monomer wie Hydroxyethylmethacrylat (HEMA) und anschließende Polymerisation.

Aufgabe war es, ein Verfahren anzugeben, welches eine einfache und exakte Dosierung von PMMA-Pulver oder ähnlicher polymerer Pulver ohne Zusatzgeräte ermöglicht. Ferner bestand eine Aufgabe darin, die Dosierung und ein Verfahren zur Herstellung von PMMA Prothesen, insbesondere von dentalen Prothesen, sowie auch für Einbettmassen und andere Anwendungen ökonomischer zu gestallten. Weiter bestand die Aufgabe darin die Mischung und die Mischungszeiten mit den Monomeren während der Verarbeitung nicht zu beeinträchtigen und möglichst auf zusätzliche Verpackungseinheiten durch übliche Konfektionierung zu verzichten. Zudem sollte das Verfahren möglichst automatisierbar sein und beim Anwender die Verfahrensschritte minimieren.

Gelöst werden konnten die gestellten Aufgaben durch die erfindungsgemäßen Formkörper nach Anspruch 1 oder 9, die quasi als vorkonfektionierte Zwischenprodukte in dreidimensionaler Form bereitgestellt werden können und im Wesentlichen die Verarbeitungseigenschaften der pulverförmigen PMMA Materialien aufweisen. Ebenso werden die Aufgaben durch das erfindungsgemäße Verfahren nach Anspruch 5 und das Kit nach Anspruch 13 sowie die Verwendung nach den Ansprüchen 10, 11 und 12 gelöst. Vorteilhafte Ausgestaltungen der Erfindung sind in den Unteransprüchen dargelegt sowie detailliert in der Beschreibung, Erfindungsgemäß wird die gewünschte Pulvermenge mit einer definierten Lösungsmittelmischung vermischt.

Gegenstand der Erfindung sind dreidimensionale Formkörper, die aus polymeren Partikeln gebildet sind, wobei die polymeren Partikel umfassen pulverförmige Partikel, Pulver, Körner, Pellets, Granulate, sphärische Partikel, Extrudate, insbesondere stäbchenförmige oder dem Fachmann bekannte Formen, und/oder Gemische verschiedener Partikel, und die Partikel an den Kontaktpunkten der polymeren Partikel untereinander zumindest teilweise miteinander fest verbunden sind, nämlich verklebt sind, und die polymeren Partikel aus organischen Polymeren bestehen, Vorzugsweise bestehen die Formkörper aus den polymeren Partikeln. In einer Alternative umfassen die Formkörper aus den polymeren Partikeln und mindestens einem Monomer umfassend die nachfolgend genannten. Gemische verschiedener Partikel können insbesondere auch Partikel gleicher oder unterschiedlicher Geometrie sein. Nach einer Alternative können vorzugsweise Partikel gleicher Geometrie aber unterschiedlicher Partikelgrößen in den Formkörper vorliegen. Bevorzugte polymere Partikel sind sphärische Partikel mit einem definierten oder mit verschiedenen Durchmessern. Sphärische Partikel sind im Wesentlichen rund. Die Partikel können auch eine Perlenform aufweisen. Besonders bevorzugte polymere sphärische Partikel haben ein Aspektverhältnis von kleiner gleich 1,8, insbesondere kleiner gleich 1,5, bevorzugt kleiner gleich 1,25, besonders bevorzugt kleiner gleich 1,1. Die Partikelgrößen liegen vorzugsweise im Bereich von 1 bis 150 µm, vorzugsweise werden verschiedene Fraktionen von Partikelgrößen miteinander gemischt. Bevorzugt können Partikel einer Fraktion von 1 bis 50 µm, insbesondere 1 bis 30 µm, und einer zweiten Fraktion von 75 bis 150 µm zu einem Formkörper verarbeitet werden. Es können auch Partikel mehrerer Fraktionen an Partikelgrößen eingesetzt werden.

Erhältlich sind die dreidimensionalen Formkörper, indem die polymeren, insbesondere pulverförmigen Partikel mit einem Lösemittel, einem Lösungsmittelgemisch optional jeweils zusammen mit einem Monomer behandelt werden, so dass lediglich die Oberflächen der Partikel benetzt werden und oberflächlich die organischen Partikel angelöst oder benetzt werden. Dies kann sich in einem leichten Anquellen der polymeren Partikel, vorzugsweise Polymerperlen an den späteren Kontaktpunkten bemerkbar machen. Daher ist ein definiertes Verhältnis von polymeren Partikeln und Lösemitteln optional zusammen mit Monomeren bei definierter Partikelgröße zu wählen, um ein zu starkes Auflösen der Partikel zu vermeiden. Das Gewichts-Verhältnis der polymeren Partikel, insbesondere der sphärischen Partikel oder Pulver, und Lösemittel optional umfassend Monomere liegt im Bereich von etwa 100 zu kleiner 20, insbesondere 100 : 15. Die Quellzeit liegt üblicherweise bei 1 bis 2 Minuten.

Die Lösemittel- und optional Monomermenge ist so auszuwählen, dass die Polymerperlen nur an den Kontaktpunkten der einzelnen Perlen leicht anquellen und verkleben und die Porosität des Pulvers und des entstehenden Formkörpers nicht wesentlich beeinträchtigt wird.

Kern der Erfindung ist, dass sich hochporöse Formkörper bilden, deren Porosität der der losen und unverdichteten polymeren Partikeln entspricht, um die Verarbeitungseigenschaften beim Nutzer im Vergleich zu den Pulvern nicht wesentlich zu verändern. Die PMMA Partikel liegen häufig in Form von Perlen mit definiertem Durchmesser vor.

Zur Herstellung der Formkörper können generell Fraktionen von Partikeln verschiedener Durchmesser eingesetzt werden. Ein Vorteil der Verwendung von verschiedenen Fraktionen der Partikel ist die Möglichkeit, die Verarbeitungszeit bei der späteren Umsetzung mit dem Monomer zur Herstellung der Teige zur Herstellung der Prothesen einstellen zu können. Besonders bewährt haben sich zur Herstellung der Formkörper Gemische aus Wasser, einer geringen Menge an einem kurzkettigen Alkohol wie z.B. Ethanol oder Methanol oder einem Keton wie z.B. Aceton oder MMA. Wässrige Gemische von Alkoholen oder Ketonen werden daher bevorzugt bei der Herstellung der Formkörper eingesetzt.

Erfindungsgemäß werden die polymeren Partikel, vorzugsweise ein PMMA-Pulver, in einem Lösungsmittel, Lösungsmittelgemisch optional enthaltend ein Monomer dispergiert und in eine Form gegossen, optional kann es auch gepresst werden.

Nach einer Einwirkzeit des Lösungsmittels auf die polymeren Partikel, vorzugsweise Polymerpulver, wird das Lösungsmittel anschließend langsam bei Raumtemperatur durch Verdunsten oder durch Verdampfen vorzugsweise bei leicht erhöhter Temperatur (z.B. 50 °C), und/oder unter Vakuum, entfernt.

Der entstandene Formkörper ist so stabil, dass er die weiteren Produktions- und Handlingsschritte, wie die Entformung, Verpackung etc. ohne mechanische Beschädigungen übersteht.

Der so hergestellte Formkörper weist eine definierte mechanische Stabilität auf. Zudem ist der Formkörper porös. Die Porosität des Formkörpers ist an die spätere Verarbeitung mit Monomeren angepasst, die von dem porösen Formkörper aufgenommen, vorzugsweise aufgesaugt werden können. Gegenstand der Erfindung ist somit ein poröser, dreidimensionaler Formkörper, der mindestens ein Monomer, vorzugsweise MMA in kurzer Zeit aufnimmt, insbesondere aufsaugt, und zu einem pastösen oder cremeartigen, gieß-, preß- oder injizierbaren Teig zerfällt.

Der erfindungsgemäße poröse Formkörper kann eine Flüssigkeit, deren Hauptbestandteil Methylmethacrylat ist, aufnehmen, insbesondere aufsaugen. Vorteilhaft enthält die Flüssigkeit zu größer 80 Gew.-% MMA, insbesondere größer gleich 90 Gew.-% MMA sowie optional Vernetzer, Initiatoren, Beschleuniger, Aktivatoren und/oder andere Hilfs- und/oder Wirkstoffe. Anschließend zerfällt der Formkörper zumindest teilweise zu den polymeren Partikeln oder ist nach kurzem Mischen, vorzugsweise Umrühren für insbesondere circa 30 Sekunden, zu einem gieß-, preß- oder injizierbaren polymeren Teig verarbeitbar.

Auf diese Weise gelingt es, die polymeren Partikel für die spätere Verarbeitung in vorkonfektionierten Dosierungen bereitzustellen, um diese zu einem Prothesenwerkstoff mit einer definierten Menge an MMA (Flüssigkeit) in kurzer Zeit, vorzugsweise innerhalb von ca. 1 bis 5 min, vorteilhaft innerhalb von 30 Sekunden, zu einem gieß-, preß- oder injizierbaren Teig zu verarbeiten. Erfindungsgemäß wird daher vorzugsweise ein Formkörper zusammen mit einer definierten Menge an Monomer in einem Kit bereitgestellt. Bevorzugt wird die benötigte Monomermenge über einen auf die Glasflasche aufgeschraubten Flaschendispenser bereitgestellt. Besonders bevorzugt umfasst das Kit eine Vielzahl an vorkonfektionierten Formkörpern sowie die entsprechend definierten Mengen an Monomer. Das Monomer kann einzeln in Vials, Phiolen abgepackt oder in einer Kartusche, die definierten Menge ausgibt in dem Kit vorliegen.

Ein besonderer Vorteil der erfindungsgemäßen Formkörper ist, dass sich ihre Verarbeitungs- und Polymerisationseigenschaften nicht wesentlich von denen der losen polymeren Partikel, insbesondere der losen Pulver unterscheiden. Die Eigenschaften der hergestellten Dentalprodukte sind wesentlich reproduzierbarer, da für jede Prothese das gleiche Mischungsverhältnis eingestellt wird, da die Materialverhältnisse immer gleich sind.

Durch eine entsprechende Dimensionierung der Formkörper können somit definierte Mengen an polymeren Partikeln in Art eines definierten "Zwischenproduktes" bereitgestellt werden. Die Formkörper können jede erdenkliche Geometrie aufweisen, bevorzugte Formen sind im Wesentlichen die Form eines Quaders, Würfels, Stabs, Zylinders, Strangs, Kugel, Kegelstumpfs, Scheibe, Rings, wabenförmig, eine rundliche konvexe Form (Eiförmig), Tetraeders oder Polyeders. Die Formkörper können einzeln oder in einer Vielzahl in einem Verpackungsmittel, wie Beutel oder in einer Schachtel abgepackt werden. Die erfindungsgemäßen Formkörper können alle eine identische oder unterschiedliche Geometrie aufweisen. Bevorzugt weisen die Formteile eine im Wesentlichen identische Masse auf.

Durch entsprechende Dimensionierung des Formkörpers bzw. Formteiles lassen sich definierte Pulvermengen dosieren, entweder durch einen Formkörper in Form eines Blocks in der entsprechenden Größe bspw. 30 g für eine dentale Totalprothese, oder mehrere gleich große Blöcke, bspw. 3 Blöcke a 10 g für eine dentale Totalprothese, oder mehrere Blöcke unterschiedlicher Größe bspw. 1 Block a 10 g und 1 Block a 5 g für eine dentale Teilprothese. Somit können durch die Bereitstellung von Formkörpern unterschiedlichen Gewichts wie 1 g, 2,5 g, 5 g, 10 g, 15 g, 20 g etc. alle gewünschten Mengen an polymeren Partikeln wie in einem Baukastensystem zusammengestellt werden. Die Mengen an Monomer werden vorzugsweise im Kit in den jeweils passenden Mengen vorkonfektioniert oder mittels einer entsprechenden Dosiervorrichtung bereitgestellt. Statt Blöcken können auch zylindrische Formkörper hergestellt werden.

Die polymeren Partikel sind erfindungsgemäß aus organischen Polymeren umfassend Acrylat- und/oder Methacrylatpolymer gebildet.

Erfindungsgemäß liegen die polymeren Partikel als Acrylat- oder Methacrylat Pulver in einer geometrisch definierten und stabilen Form als Formkörper vor und werden zur Herstellung von Prothesenwerkstoffen verwendet.

Ebenfalls Gegenstand der Erfindung ist ein dreidimensionaler Formkörper aus polymeren Partikeln, insbesondere sphärischen Partikeln, wobei die Dichte der Formkörper der Dichte der losen Partikelschüttung in etwa ähnlich oder vorzugsweise nahezu gleich ist. So kann die Dichte einer Partikelschüttung 1,1997 g/cm³ betragen und die Dichte des daraus hergestellten Formkörpers bei 1,1978 g/cm³ liegen. Die erfindungsgemäßen Formkörper weisen daher vorzugsweise eine Dichte von kleiner gleich 1,5 g/cm³ auf, insbesondere kleiner 1,4 g/cm³, kleiner gleich 1,3 g/cm³, besonders bevorzugt kleiner gleich 1,2 g/cm³. Vorteilhaft bleibt auch die Porosität der Schüttung der Partikel im Wesentlichen im Festkörper erhalten. Die polymeren Partikel umfassen pulverförmige Partikel, Pulver, Körner, sphärische Partikel, insbesondere mit einem Aspektverhältnis von kleiner gleich 1,4, Pellets, Granulate, Extrudate und/oder Gemische verschiedener Partikel, und die Partikel in den Formkörpern an den Kontaktpunkten der polymeren Partikel untereinander zumindest teilweise miteinander fest verbunden sind, nämlich verklebt sind, und die polymeren Partikel organische Polymere wie Acrylat- und/oder Methacrylatpolymer umfassen. Im Sinne der Erfindung sind insbesondere Formkörper mit einer Porosität zu größer gleich 95%, 90 %, 85 %, 80 %, 75 % oder 70 % der Porosität der losen polymeren Partikel bei gleicher Form und Partikelgröße. Die erfindungsgemäße Porosität kann mittelbar über die jeweilige spezifische Oberfläche der losen Partikel und der Formkörper ermittelt werden.

Ebenso im Sinne der Erfindung sind Formkörper deren spezifische Oberfläche größer gleich 70 %, insbesondere größer gleich 75 %, vorzugsweise größer gleich 80%, bevorzugt größer gleich 85%, besonders bevorzugt größer gleich 90% oder größer gleich 95% der spezifischen Oberfläche der polymeren Partikel beträgt, insbesondere der losen, schüttfähigen polymeren Partikel, aus denen der Formkörper hergestellt wurde, d.h. der polymeren Partikel, die nicht an den Kontaktpunkten miteinander verbunden sind.

Entsprechend der Erfindung werden Formkörper offenbart, die porös sind und die Porosität der Formkörper derart ist, dass die Formkörper Methylmethacrylat n kurzer Zeit aufnehmen, insbesondere aufsaugen. Ferner zerfallen die Formkörper zumindest teilweise, vorzugsweise vollständig zu den polymeren Partikeln oder zu einem gieß-, preß- oder injizierbaren polymeren Teig, der über einen bestimmten Zeitraum verarbeitbar ist. Vorzugsweise ist der Formkörper innerhalb von 2 Minuten nach dem Inkontaktbringen mit dem Monomer zu einem gieß-, preß-oder injizierbaren Teig verarbeitbar.

Erfindungsgemäß liegen die Formkörper in einer geometrisch definierten dreidimensionalen Form vor und sind insbesondere dimensionsstabil gegenüber einer Druckbelastung (Druckfestigkeit größer gleich 1 MPa, vorzugsweise größer gleich 1,5 MPa, bevorzugt größer gleich 2,0 MPa, besonders bevorzugt größer gleich 2,25 MPa. Vorteilhaft bis 2,45 MPa oder größer als 2,45 MPa. Zusätzlich oder alternativ weisen die Formkörper insbesondere eine diametrale Zugfestigkeit von größer gleich 0,1 MPa auf, insbesondere größer gleich 0,2 MPa, bevorzugt größer gleich 0,3 MPa, besonders bevorzugt größer gleich 0,4 MPa, größer gleich 0,44 MPa. Die erfindungsgemäßen hergestellten Formkörper wiesen eine Druckfestigkeit von 2,45 MPa auf. Ihre diametrale Zugfestigkeit lag bei 0,44 MPa. Die Druckfestigkeit wird nach DIN EN ISO 9917-1 (Anhang D) bestimmt und die diametrale Zugfestigkeit nach ADA-Specification No. 27.

Erfindungsgemäße Formkörper umfassen polymere Partikel umfassend Polymere, wie Homo- und/oder Co-Polymere, basieren auf mindestens einem der Monomere, umfassend eine (Meth-)acrylat-Gruppe ausgewählt aus Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, eine Mischung enthaltend mindestens eines dieser (Meth-)acrylate und/oder Co-Polymere umfassend eines oder mindestens zwei der vorgenannten Monomere. Darüber hinaus können die Polymere auch Gemische dentaler Monomeren wie z.B. MMA und zusätzlich mindesten einen Vernetzer umfassen. Typische Vernetzer sind BDMA, 1,4-Butandiol-dimethacrylat (1,4-BDMA) oder Pentaerythritol-tetraacrylat, Urethandimethacrylat (UDMA), Bis-GMA-Monomer (Bisphenol-A-Glycidyl-Methacrylat). Die Verwendung von Verdünnungsmitteln (dünnflüssige Acrylate wie Triethylenglycoldimethacrylat (TEGDMA) und Diethylenglycoldimethacrylat (DEGMA), usw. Weitere Vernetzer sind nachfolgend auch unter den polymeren Partikeln umfassend Co-Polymere offenbart, die mindesten ein (Meth-)acrylat Monomer mit zwei, drei, vier, fünf oder sechs (Meth-)acrylat-Gruppen umfassen.

Ebenfalls im Sinne der Erfindung sind polymere Partikel, die auf mindestens einem (Meth-)-acrylat Monomer mit nur einer (Meth-)acrylat-Gruppe basieren oder die auf der Mischung mindestens zweier dieser (Meth-)acrylat Monomere basiert.

Besonders bevorzugte Formkörper nach der Erfindung zeichnen sich dadurch aus, dass der Formkörper an den Kontaktpunkten, an denen die polymeren Partikel untereinander zumindest teilweise miteinander fest verbunden sind, Monomere enthält, insbesondere sind sie an den Kontakpunkte mit Monomeren verklebt, und die Monomere umfassen (Meth-)acrylat mit einer (Meth-)acrylat-Gruppe ausgewählt aus Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat und/oder eine Mischung enthaltend mindestens eines dieser (Meth-)acrylate.

Zur Herstellung der Knochenzemente, Einbettmassen etc. werden die Formkörper mit einem härtbaren Monomer, wie Acrylat- oder Methacrylat oder deren Mischung in Kontakt gebracht, vorzugsweise getränkt und entsprechend verarbeitet, vorzugsweise in Form gebracht. Nachfolgend kann die Härtung erfolgen. Gehärtet werden kann durch Selbsthärtung, Strahlenhärtung, insbesondere Lichthärtung und/oder durch thermische Härtung. Beispielsweise kann durch Belichtung mit UV-Licht und/oder thermisch durch Erhitzen erfolgen. Zusammen mit den Monomeren oder separat können auch übliche Photoinitiatoren, Aktivatoren, Stabilisatoren, heißhärtende Initiatoren sowie weitere übliche Zusatzstoffe oder Hilfsstoffe.

Eine weitere Ausführungsform der Erfindung betrifft ein Verfahren zur Herstellung des Formkörpers sowie einen Formkörper erhältlich nach dem Verfahren, indem
(i). polymere Partikel organischer Polymere wie vorstehend angegeben,
(ii). mit einem Lösemittel oder Lösungsmittelgemisch jeweils optional umfassend mindestens ein Monomer behandelt werden unter Erhalt einer Mischung,
(iii). Formung der Mischung,
(iv). Entfernen des Lösemittels oder Lösemittelgemisches.
(v). Erhalt des Formkörpers.

Erfindungsgemäß wird in (ii). ein Monomer, in dem die polymeren Partikel löslich sind vorzugsweise mit einem Lösemittel oder Lösemittelgemisch, in dem die polymeren Partikel ebenfalls löslich sind, und die flüchtig sind, eingesetzt. Alternativ ist es erfindungsgemäß ebenfalls bevorzugt ein Alkohol/Wasser oder ein Keton/Wasser-Gemisch einzusetzen. Vorzugsweise werden in (ii) als Lösemittel oder Lösungsmittelgemisch jeweils optional umfassend mindestens ein Monomer, mindestens ein kurzkettiger Alkohol mit 1 bis 4 C-Atomen, wie Methanol, Ethanol, oder ein Keton, wie Aceton, ein wässriges Gemisch eines vorgenannten Alkohols oder Ketons, mindestens ein Monomer umfassend ein (Meth-)-acrylat mit einer (Meth-)acrylat-Gruppe oder ein Gemisch des Monomers mit mindestens einem Alkohol oder Keton eingesetzt.

Zur Verklebung oder zum Anlösen der polymeren Partikel kann vorzugsweise in (ii) ein Monomer verwendet werden, dass ein (Meth-)acrylat mit einer (Meth-)acrylat-Gruppe umfasst, und das ausgewählt ist aus Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat und/oder eine Mischung enthaltend mindestens eines dieser (Meth-)acrylate.

Nach einer bevorzugten Variante wird in dem Verfahren als (ii) Lösemittel oder Lösungsmittelgemisch jeweils optional umfassend mindestens ein Monomer eingesetzt, umfassend: a) mindestens einen Alkohol umfassend Methanol und/oder Ethanol; b) mindestens ein Keton umfassend Aceton; c) mindestens ein Monomer umfassend ein (Meth-)acrylat mit einer (Meth-)acrylat-Gruppe optional im Gemisch mit mindestens einem Alkohol, oder mindestens ein Monomer umfassend ein (Meth-)acrylat mit einer (Meth-)acrylat-Gruppe optional im Gemisch mit mindestens einem Keton, bevorzugt ein Monomer wie MMA im Gemisch mit mindestens einem Keton, vorzugsweise Aceton, oder d) wässriges Gemisch von Methanol oder Ethanol. Erfindungsgemäß wird MMA im Gemisch mit mindestens einem Alkohol, vorzugsweise Ethanol eingesetzt.

Bevorzugt wird das Lösemittel oder Lösungsmittelgemisch jeweils optional umfassend mindestens ein Monomer im Gewichtsverhältnis von 1 : 200 bis 50 zu 100 eingesetzt, insbesondere 2 : 100 bis 15 : 100. Eine besonders bevorzugte Mischung, mit der sehr stabile Formkörper erhalten werden umfasst 4 bis 10 Gewichtsteile eines Gemisches von Ethanol und Monomer, wie MMA, zu etwa 100 Gewichtsteilen polymere Partikel.

Weiter bevorzugt umfasst das Gemisch aus Lösemittel und Monomer 1 bis 30 Gewichtsteile Lösemittel zu 1 bis 5 Gewichtsteilen Monomer, insbesondere 5 bis 20 Gewichtsteile Lösemittel auf 1 bis 5 Gewichtsteile Monomer. Erfindungsgemäß werden etwa 14 Gewichtsteile Lösemittel auf etwa ein Teil Monomer eingesetzt.

Zur Herstellung der Formkörper wird das Verfahren in den Schritten (ii) und/oder (iii) durchgeführt, indem das Lösemittel oder Lösungsmittelgemisch die polymeren Partikel oberflächlich benetzt und optional die Polymere anlöst. Nach der Benetzung und einem teilweisen Anlösen wird das Lösemittel oder Lösemittelgemisch verflüchtigt und der Formkörper somit getrocknet, wobei er seine Stabilität erhält.

Die nach dem erfindungsgemäßen Verfahren erhältliche Mischung wird im Schritt (iii) zur Ausbildung des Formkörpers in eine Form überführt, insbesondere bildet sich der Grünling aus dem durch Trocknung der Formkörper gebildet wird. Erfindungsgemäß wird in (iii). die Mischung in eine Form überführt, insbesondere ist die Form ein Negativ eines Quaders, Würfels, Stabs, Zylinders, Strangs, Kugel, einer rundlichen konvexen Form (eiförmig), Tetraeders oder Polyeders, vorzugsweise ist die Form jeweils einseitig offen bzw. zu öffnen. Zur Ausbildung des Formkörpers können die vorgenannten Formen ein-, zwei oder mehrteilig ausgebildet sein. Sofern notwendig kann die Mischung in der Form mit definiertem Druck gepresst werden, um die Kontaktpunkte der polymeren Partikel ausreichend zusammenzupressen, um bei der nachfolgenden Trocknung eine ausreichende Verbindung zwischen den Kontaktpunkten sicherzustellen.

Zur Trocknung wird in (iv). das Lösemittel oder Lösungsmittelgemisch vorzugsweise durch Verdunsten, Zuführen von Wärme optional im Vakuum entfernt. Besonders geeignet ist die Trocknung oder Verflüchtigung der Lösemittel bei Temperaturen zwischen 20 bis 60 °C, vorzugsweise um etwa 50 °C optional im Vakuum.

Ebenso Gegenstand der Erfindung ist die Verwendung der Formkörper zur reproduzierbaren Dosierung der polymeren Partikel. Die erfindungsgemäßen Formkörper erlauben eine reproduzierbare und sehr genaue Dosierung für eine Vielzahl an Anwendungen ohne, dass eine separate Verpackung notwendig ist. Bevorzugt werden die Formkörper zur Dosierung der polymeren Partikel bei der Herstellung von Dentalprothesen, Einbettmassen in der Histologie, Metallographie, Veterinärmedizin verwendet.

Ferner ist Gegenstand der Erfindung die Verwendung der Formkörper zusammen mit mindestens einem Monomer umfassend (Meth-)acrylat mit einer (Meth-)acrylat-Gruppe umfassend Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat und/oder eine Mischung enthaltend mindestens eines dieser (Meth-)acrylate zur Herstellung eines pastösen oder cremeartigen, gieß-, preß- oder injizierbaren polymeren Teigs, der strahlen- und/oder thermisch härtbar oder selbsthärtend ist.

Besonders bevorzugt werden die Formkörper verwendet zur Dosierung der polymeren Partikel bei der Herstellung von Prothesen, Dentalprothesen, Prothesenwerkstoffen, Einbettmassen in der Histologie, Metallographie, Knochenzementen, Prothese in der Veterinärmedizin, Einbettmasse für ein poröses Substrat, bei der Metallographie zur Präparation des Gefüges eines Substrates, für transparente Schliffeinbettung in der Materialprüfung, als Einbettmasse für die Prüfung von Leiterplatten, als Einbettmasse für die Prüfung elektronischer Bauteile, als Einbettmasse für die Prüfung in der Halbleitertechnik, als Einbettmasse für die Prüfung mikroelektronischer Bauteile, als Einbettmasse für die Prüfung in der Optoelektronik, als Einbettmasse für die Prüfung in der medizinischen Gerätetechnik und/oder als Einbettmasse für die Prüfung medizinischer Instrumenten, als Einbettmasse bzw. Einbettmaterial, bei einer Materialprüfung eines Substrates.

Nach einer weiteren Ausführungsform ist Gegenstand der Erfindung ein Kit, aufweisend mindestens einen Formkörper (a), insbesondere eine Vielzahl an Formkörpern, und davon getrennt mindestens eine definierte Menge mindestens eines vorkonfektionierten polymerisierbaren Monomers (b), wobei
(a) der mindestens eine erfindungsgemäßen Formkörper aus
(a1) mindestens einem in (b) löslichen organischen Polymer wie vorstehend angegeben gebildet ist, und
(b) mindestens eine definierte Menge eines radikalisch polymerisierbaren Monomers umfassend (Meth-)acrylat mit einer (Meth-)acrylat-Gruppe umfasst, insbesondere ausgewählt aus Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat und/oder eine Mischung enthaltend mindestens eines dieser (Meth-)acrylate, optional umfasst das Kit (c) Photoinitiator und gegebenenfalls ein Gehalt an Aktivator und optional (d) mindestens ein Photoinitiator und/oder mindestens einen heiß- oder selbsthärtenden Initiator.

Das Monomer (b) im Kit dient der Herstellung der Teige, wie Knochenzemente oder Dentalprothesen. Vorzugsweise ist das Monomer definiert portioniert oder portionierbar, um reproduzierbare Mischungen aus (a) und (b) herzustellen.

Ebenfalls können die polymeren Partikel Co-Polymere mit mindestens zwei verschiedenen (Meth-)acrylat-Gruppen umfassen, wobei ein Co-Monomer basiert auf mindestens einem (Meth-)acrylat Monomer mit zwei, drei, vier, fünf (Meth-)acrylat-Gruppen und/oder sechs (Meth-) acrylat-Gruppen oder eine Mischung mindestens zweier dieser (Meth-)acrylate.

Nachfolgend sind geeignete Co-Monomere genannt, die in Co-Polymeren und/oder als Monomer oder Co-Monomer in (ii) sowie optional bei der späteren Verarbeitung der Formkörper zu Prothesen oder Einbettmassen eingesetzt werden können: (Meth-)acrylat mit mindestens zwei (Meth-)acrylat-Gruppen ausgewählt aus Ethandioldimethacrylat, Tetraethylenglykoldimethacrylat, Diethylenglykoldimethacrylat, Ethylenglykoldimethacrylat, Polyethylenglykoldimethacrylat (400) oder (600), Butandioldimethacrylat, Hexandioldimethacrylat, Decandioldimethacrylat, Dodecandioldimethacrylat, 1,3-Butylenglykoldimethacrylat, Dipropylenglykolmethacrylat, Bisphenol-A-dimethacrylat, Bisphenol-A-dimethacrylat Derivat, wie ethoxyliertes 2-Bisphenol-A-dimethacrylat, Trimethylolpropantrimethacrylat, Triethylenglykoldimethacrylat, 2,2-Bis-4-(3-methacryloxy-2-hydroxy-propoxy)-phenylpropan (Bis-GMA), Tricyclodecandimethanoldimethacrylat, ein Urethanmethacrylat mit mindestens zwei Methacrylat-Gruppen, Ethandioldiacrylat, Tetraethylenglykoldiacrylat, Diethylenglykoldiacrylat, Ethylenglykoldiacrylat, Polyethylenglykoldiacrylat (400) oder (600), Butandioldiacrylat, Hexandioldiacrylat, Decandioldiacrylat, Dodecandioldiacrylat, 1,3-Butylenglykoldiacrylat, Dipropylenglykolacrylat, Bisphenol-A-diacrylat, Bisphenol-A-diacrylat Derivat, wie ethoxyliertes 2-Bisphenol-A-diacrylat, Trimethylolpropantriacrylat, Triethylenglykoldiacrylat, 2,2-Bis-4-(3-methacryloxy-2-hydroxy-propoxy)-phenylpropan (Bis-GMA), Tricyclodecandimethanoldiacrylat und/oder ein Urethanacrylat mit mindestens zwei Acrylat-Gruppen oder einer Mischung enthaltend mindestens eines der (Meth-)acrylate.

Weitere geeignete Co-Monomere, die in Co-Polymeren und/oder als Monomer oder Co-Monomer in (ii) sowie optional bei der späteren Verarbeitung der Formkörper zu Prothesen oder Einbettmassen eingesetzt werden können sind: (Meth-)acrylat mit drei bis sechs (Meth-)acrylat-Gruppen ausgewählt aus (i) mit drei (Meth-)acrylat-Gruppen aus ethoxyliertem-(15)-Trimethylolpropan-Triacrylat, ethoxyliertem-5-Pentaerythritoltriacrylat, propoxyliertes-(5.5)-Glyceryltriacrylat, Trimethylolpropantrimethacrylat, Tris(2-hydroxyethyl)-isocyanurat-triacrylat, und/oder (ii) mit vier (Meth-)acrylat-Gruppen aus Di-Trimethylolpropan-tetra-acrylat, ethoxyliertes-(4)-Pentaerythritol-tetra-acrylat, Pentaerythritol-tetra-acrylat, Di-Trimethylolpropan-tetra-methacrylat, ethoxyliertes-(4)-Pentaerythritol-tetra-methacrylat, Pentaerythritol-tetramethacrylat und/oder (iii) mit fünf (Meth-)acrylat-Gruppen aus Di-Pentaerythritol-pentaacrylat, i-Pentaerythritol-pentamethacrylat, Dipentaerythritol pentaacrylate, Di(tetramethylolmethan)-pentamethacrylat und/oder (iv) mit sechs (Meth-)acrylat-Gruppen ein Dipentaerythrit-hexa(meth)acrylat. Ebenfalls geeignet sind Oligomere von (Meth-)acrylaten, insbesondere Urethan-di-Acrylat-Oligomer.

Nachfolgend wird die Erfindung anhand von Beispielen näher erläutert, ohne sie auf die Beispiele zu beschränken:

### Ausführungsbeispiel:

Herstellung Mischung: Aus 6,5 ml Ethanol und 0,5 ml Methylmethacrylat wird bei Raumtemperatur eine Mischung hergestellt.

Herstellung Dispersion: 100 g PMMA-Pulver (z.B. PalaXpress) werden mit der oben beschriebenen Mischung bei Raumtemperatur versetzt und ca. 2 min. intensiv durch Rühren vermischt.

Herstellung Formteil: Die Dispersion wird in eine Form mit der gewünschten Geometrie gepresst. Alternativ kann eine Suspension in eine dreidimensionale Form gegossen werden.

Typischerweise besteht die Form aus Kunststoff und ist beständig gegenüber den eingesetzten Lösungsmitteln bei den entsprechenden Temperaturen. Bevorzugte Formen werden aus flexiblen oder elastischen Kunststoffen hergestellt. Als besonders geeignet haben sich dreidimensionale Silikon-Formen erwiesen, da diese sich besonders gut entformen lassen. Generell können auch mehrteilige, wie zweiteilige Metallformen oder mehrteilige nicht flexible Formen aus anderen Materialien verwendet werden. Die verwendbaren Formen sind nicht auf die genannten Beispiele beschränkt, sondern können generell zur Herstellung erfindungsgemäßer Formkörper verwendet werden.
Die Mischung kann in eine zylindrische Kunststoffform gestrichen werden. Nach erfolgter Trocknung können zylindrische Probekörper von ca. 30 g (Höhe 35 mm, Durchmesser 40 mm) entnommen werden. Die Druckfestigkeit von erfindungsgemäßen Formkörpern wurde mit 2,45 MPa gemessen. Die diametrale Zugfestigkeit wurde mit 0,44 MPa bestimmt. Die Druckfestigkeit wird nach DIN EN ISO 9917-1 (Anhang D) bestimmt und die diametrale Zugfestigkeit nach ADA-Specification No. 27.

Trocknen: Man lässt entweder bei Raumtemperatur oder bei leicht erhöhten Temperaturen (ca. 45 °C) das Lösungsmittelgemisch langsam verdunsten. Nachdem das Lösungsmittel vollständig oder nahezu vollständig verdunstet ist erreicht der Formkörper seine mechanische und geometrische Stabilität.

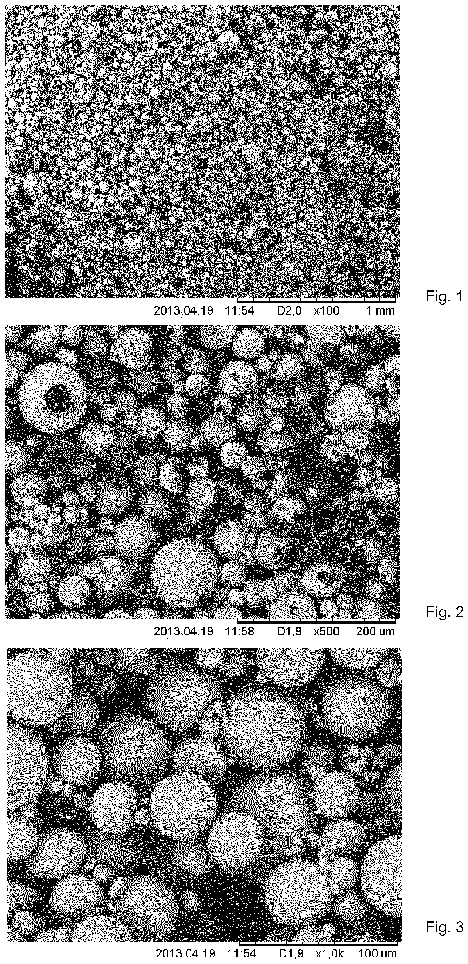

Ggf. kann der Formkörper nach dem Entformen durch leichtes Erwärmen im Vakuum nochmals nachgetrocknet werden. Die Figuren 1 bis 5 zeigen REM-Aufnahmen der erfindungsgemäßen

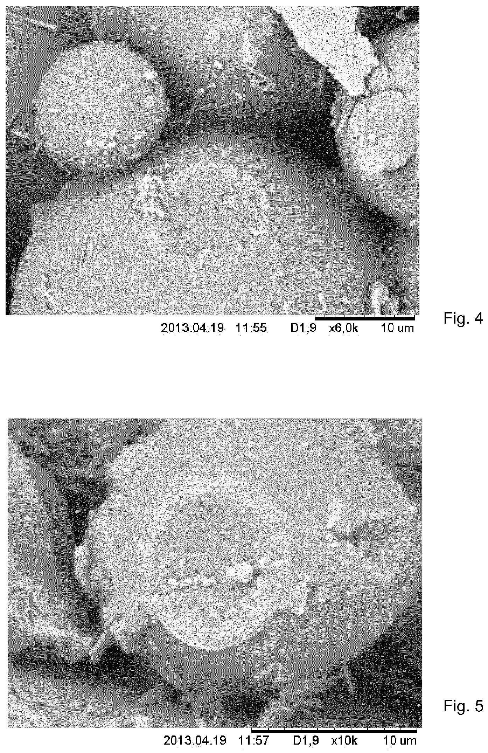

Formkörper in unterschiedlicher Auflösung. Auflösung Figur 1 (1 mm), Figur 2 (200 Mikrometer, µm), Figur 3 (100 Mikrometer), Figur 4 (10 Mikrometer), Figur 5 (10 Mikrometer). Die Kontaktpunkte der polymeren Partikel der Formkörper sind besonders gut in den Figuren 4 und 5 an den flachen Oberflächen der sphärischen Partikel (Perlen) zu erkennen. Figur 4 zeigt eine REM-Aufnahme eines Formkörpers mit kleine und großen sphärischen Partikeln. Der Erhalt der Porosität in den Formkörpern ist in den Figuren 1 bis 5 gut zu erkennen.

## Patentansprüche

1. Formkörper zur reproduzierbaren Dosierung polymerer Partikel, der ein dreidimensionaler, poröser Formkörper gebildet aus polymeren Partikeln ist, **dadurch gekennzeichnet, dass** die polymeren Partikel Pulver, Körner, Pellets, Granulate, sphärische Partikel, Extrudate und/oder Gemische verschiedener Partikel umfassen, und die Partikel an den Kontaktpunkten der polymeren Partikel untereinander zumindest teilweise miteinander fest verbunden sind, wobei die Partikel verklebt sind, und die polymeren Partikel aus organischen Polymeren bestehen, wobei die organischen Polymere ausgewählt sind aus Acrylat- und/oder Methacrylatpolymer, PMMA Poly(methyl-2-methylpropenoat), Polymeren basierend auf Monomeren, umfassend mindestens eine (Meth-)acrylat-Gruppe ausgewählt aus Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykol-monomethacrylat, Tetrahydrofuryl-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylenglykol-monoacrylat, Tetrahydrofuryl-acrylat, Hydroxyethylacrylat, Hydroxypropylacrylat, Hydroxyethylmethacrylat, Hydroxypropylmethacrylat, einer Mischung enthaltend mindestens eines dieser (Meth-)acrylate und/oder Co-Polymere enthaltend mindestens eines oder mindestens zwei der genannten Monomere und der Formkörper in einer geometrisch definierten dreidimensionalen Form vorliegt, und wobei die Porosität des Formkörpers derart ist, dass der Formkörper eine Flüssigkeit, deren Hauptbestandteil Methylmethacrylat ist, aufnimmt, insbesondere aufsaugt, und der Formkörper zumindest teilweise zu den polymeren Partikeln zerfällt oder nach kurzem Mischen zu einem gieß-, preß- oder injizierbaren polymeren Teig verarbeitbar ist, erhältlich durch ein Verfahren, in dem die polymeren Partikel mit einem Lösemittel, einem Lösungsmittelgemisch optional jeweils zusammen mit einem Monomer behandelt werden, so dass lediglich die Oberflächen der Partikel benetzt werden und oberflächlich die organischen Partikel angelöst oder benetzt werden.

2. Formkörper nach Anspruch 1 , **dadurch gekennzeichnet, dass** der Formkörper an den Kontaktpunkten an denen die polymeren Partikel untereinander zumindest teilweise miteinander fest verbunden sind, Monomere enthält umfassend (Meth-)acrylat mit einer (Meth-)acrylat-Gruppe ausgewählt aus Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykol-monomethacrylat, Tetrahydrofuryl-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylenglykol-monoacrylat, Tetrahydrofuryl-acrylat, Hydroxyethylacrylat, Hydroxy-propylacrylat, Hydroxyethylmethacrylat, Hydroxypropylmethacrylat und/oder eine Mischung enthaltend mindestens eines dieser (Meth-)acrylate.

3. Formkörper nach einem der Ansprüche 1 oder 2, **dadurch gekennzeichnet, dass** die spezifische Oberfläche des Formkörpers mindestens 80% der spezifischen Oberfläche der polymeren Partikel beträgt aus denen der Formkörper hergestellt wurde.

4. Formkörper nach einem der Ansprüche 1 bis 3, **dadurch gekennzeichnet, dass** der Formkörper im Wesentlichen die Form eines Quaders, Würfels, Stabs, Zylinders, Strangs, Kugel, rundliche konvexe Form, Tetraeders oder Polyeders aufweist.

5. Verfahren zur Herstellung des Formkörpers zur reproduzierbaren Dosierung polymerer Partikel nach einem der Ansprüche 1 bis 4, in dem
(i). polymere Partikel organischer Polymere, wobei die polymeren Partikel aus organischen Polymeren bestehen, wobei die organischen Polymere ausgewählt sind aus Acrylat- und/oder Methacrylatpolymer, PMMA Poly(methyl-2-methylpropenoat), Polymeren basierend auf Monomeren, umfassend mindestens eine (Meth-)acrylat-Gruppe ausgewählt aus Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykol-monomethacrylat, Tetrahydrofuryl-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylenglykol-monoacrylat, Tetrahydrofuryl-acrylat, Hydroxyethylacrylat, Hydroxy-propylacrylat, Hydroxyethylmethacrylat, Hydroxypropylmethacrylat, einer Mischung enthaltend mindestens eines dieser (Meth-)acrylate und/oder Co-Polymere enthaltend mindestens eines oder mindestens zwei der genannten Monomere,
(ii). mit einem Lösemittel oder Lösungsmittelgemisch jeweils optional umfassend mindestens ein Monomer behandelt werden unter Erhalt einer Mischung,
(iii). Formung der Mischung,
(iv). Entfernen des Lösemittels oder Lösemittelgemisches,
(v). Erhalt des Formkörpers.

6. Verfahren nach Anspruch 5, **dadurch gekennzeichnet, dass** das (ii) Lösemittel oder Lösungsmittelgemisch jeweils optional umfassend mindestens ein Monomer umfasst mindestens einen kurzkettigen Alkohol mit 1 bis 4 C-Atomen, wie Methanol, Ethanol, Keton, wie Aceton, ein wässriges Gemisch eines vorgenannten Alkohols oder Ketons, optional mindestens ein Monomer umfassend ein (Meth-)acrylat mit einer (Meth-)acrylat-Gruppe oder ein Gemisch des Monomers mit mindestens eines Alkohol oder Keton.

7. Verfahren nach Anspruch 6, **dadurch gekennzeichnet, dass** das (ii) Lösemittel oder Lösungsmittelgemisch jeweils optional umfassend mindestens ein Monomer umfasst
a) mindestens einen Alkohol umfassend Methanol und/oder Ethanol,
b) mindestens ein Keton umfassend Aceton,
c) mindestens ein Monomer umfassend ein (Meth-)acrylat mit einer (Meth-)acrylat-Gruppe optional im Gemisch mit mindestens einem Alkohol und/oder Keton oder
d) wässriges Gemisch von Methanol oder Ethanol oder
e) wässriges Gemisch mit Aceton.

8. Verfahren nach einem der Ansprüche 5 bis 7, **dadurch gekennzeichnet, dass** in (iii). die Mischung in eine Form überführt wird, insbesondere weist die Form die Form eines Quaders, Würfels, Stabs, Zylinders, Strangs, Kugel, rundliche konvexe Form, Tetraeders oder Polyeders auf.

9. Formkörper erhältlich nach einem Verfahren nach einem der Ansprüche 5 bis 8.

10. Verwendung eines Formkörpers nach einem der Ansprüche 1 bis 4 oder 9 oder erhalten nach einem der Ansprüche 5 bis 8 zur reproduzierbaren Dosierung der polymeren Partikel.

11. Verwendung eines Formkörpers nach einem der Ansprüche 1 bis 4 oder 9 oder erhalten nach einem der Ansprüche 5 bis 8 zusammen mit mindestens einem Monomer umfassend (Meth-)acrylat mit einer (Meth-)acrylat-Gruppe umfassend Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykol-monomethacrylat, Tetrahydrofuryl-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylenglykol-monoacrylat, Tetrahydrofuryl-acrylat, Hydroxyethylacrylat, Hydroxypropylacrylat, Hydroxyethylmethacrylat, Hydroxypropylmethacrylat und/oder eine Mischung enthaltend mindestens eines dieser (Meth-)acrylate zur Herstellung eines pastösen, gieß- oder injizierbaren polymeren Teigs, der strahlen- und/oder thermisch härtbar ist.

12. Verwendung eines Formkörpers nach Anspruch 1 bis 4 oder 9 zur Dosierung der polymeren Partikel bei der Herstellung von Prothesen, Dentalprothesen, Prothesenwerkstoffen, Einbettmassen in der Histologie, Metallographie, Knochenzementen, Prothese in der Veterinärmedizin, von Spacern, Einbettmasse für ein poröses Substrat, bei der Metallographie zur Präparation des Gefüges eines Substrates, für transparente Schliffeinbettung in der Materialprüfung, als Einbettmasse für die Prüfung von Leiterplatten, als Einbettmasse für die Prüfung elektronischer Bauteile, als Einbettmasse für die Prüfung in der Halbleitertechnik, als Einbettmasse für die Prüfung mikroelektronischer Bauteile, als Einbettmasse für die Prüfung in der Optoelektronik, als Einbettmasse für die Prüfung in der medizinischen Gerätetechnik und/oder als Einbettmasse für die Prüfung medizinischer Instrumente, als Einbettmasse bzw. Einbettmaterial, bei einer Materialprüfung eines Substrates.

13. Kit, aufweisend mindestens einen Formkörper (a) und davon getrennt mindestens eine definierte Menge mindestens eines vorkonfektionierten polymerisierbaren Monomers (b), wobei
(a) der mindestens eine Formkörper nach einem der Ansprüche 1 bis 4 oder 9 oder erhalten nach einem der Ansprüche 5 bis 8 aus
(a1) mindestens einem in (b) löslichen organischen Polymer ausgewählt aus Acrylat- und/oder Methacrylatpolymer, PMMA Poly(methyl-2-methylpropenoat), Polymeren basierend auf Monomeren, umfassend mindestens eine (Meth-)acrylat-Gruppe ausgewählt aus Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykol-monomethacrylat, Tetrahydrofurylmethacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylenglykol-monoacrylat, Tetrahydrofuryl-acrylat, Hydroxyethylacrylat, Hydroxy-propylacrylat, Hydroxyethylmethacrylat, Hydroxypropylmethacrylat, einer Mischung enthaltend mindestens eines dieser (Meth-)acrylate und/oder Co-Polymere enthaltend mindestens eines oder mindestens zwei der genannten Monomere, gebildet ist, und
(b) mindestens eine definierte Menge eines radikalisch polymerisierbaren Monomers umfassend eine (Meth-)acrylat-Gruppe.

14. Kit nach Anspruch 13, **dadurch gekennzeichnet, dass** (b) die mindestens eine definierte Menge eines radikalisch polymerisierbaren Monomers ausgewählt ist aus Acrylat, Methylmethacrylat, Ethylenmethacrylat, Propylmethacrylat, Butylmethacrylat, n-Hexylmethacrylat, 2-Phenoxyethylmethacrylat, Isobornylmethacrylat, Isodecylmethacrylat, Polypropylenglykol-monomethacrylat, Tetrahydrofuryl-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylenglykol-monoacrylat, Tetrahydrofuryl-acrylat, Hydroxyethylacrylat, Hydroxypropylacrylat, Hydroxyethylmethacrylat, Hydroxypropylmethacrylat und/oder einer Mischung enthaltend mindestens eines dieser (Meth-)-acrylate, optional umfasst das Kit
(c) Photoinitiator und gegebenenfalls einen Gehalt an Aktivator und optional
(d) mindestens einen Photoinitiator und/oder mindestens einen heiß- oder selbsthärtenden Initiator.

## Claims

1. Moulded body for reproducible dosing of polymeric particles, being a three-dimensional porous moulded body formed from polymeric particles, **characterised in that** the polymeric particles comprise powders, grains, pellets, granulates, spherical particles, extrudates and/or mixtures of different particles, and the particles are firmly connected to each other, at least in part, at the contact points of the polymeric particles, the particles being glued together, and the polymeric particles consisting of organic polymers, the organic polymers being selected from acrylate and/or methacrylate polymer, PMMA poly(methyl-2-methylpropenoate), polymers based on monomers comprising at least one (meth-)acrylate group selected from methyl methacrylate, ethylene methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethylene acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate, a mixture containing at least one of said (meth-)acrylates and/or co-polymers containing at least one or at least two of the afore-mentioned monomers, and the moulded body being present in a geometrically defined three-dimensional shape, and the porosity of the moulded body being such that the moulded body absorbs, in particular sponges, a liquid, the main component of which being methyl methacrylate, and the moulded body disintegrating, at least in part, into the polymeric particles or being processible to a castable, pressable or injectable polymeric dough after short mixing, obtainable by a method in which the polymeric particles are treated with a solvent, a solvent mixture, each optionally together with a monomer so that only the surfaces of the particles are wetted and the organic particles are superficially solvated or wetted.

2. Moulded body according to claim 1, **characterised in that** the moulded body contains monomers at the contact points at which the polymeric particles are connected to each other, at least in part, comprising (meth-)acrylate with a (meth-)acrylate group selected from acrylate, methyl methacrylate, ethylene methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethylene acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate and/or a mixture containing at least one of said (meth-)acrylates.

3. Moulded body according to any one of the claims 1 or 2, **characterised in that** the specific surface of the moulded body amounts to at least 80 % of the specific surface of the polymeric particles from which the moulded part has been produced.

4. Moulded body according to any one of the claims 1 to 3, **characterised in that** the moulded body substantially takes the shape of a cuboid, cube, rod, cylinder, strand, sphere, roundish convex shape, tetrahedron or polyhedron.

5. Method for the production of the moulded body for reproducible dosing of polymeric particles according to any one of the claims 1 to 4, in which
(i). polymeric particles of organic polymers, the polymeric particles consisting of organic polymers, the organic polymers being selected from acrylate and/or methacrylate polymer, PMMA poly(methyl-2-methylpropenoate), polymers based on monomers comprising at least one (meth-)acrylate group selected from methyl methacrylate, ethylene methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethylene acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate, a mixture containing at least one of said (meth-)acrylates and/or co-polymers containing at least one or at least two of the afore-mentioned monomers, are treated
(ii). with a solvent or solvent mixture, each optionally comprising at least one monomer to obtain a mixture,
(iii). moulding the mixture,
(iv). removing the solvent or solvent mixture,
(v). obtaining the moulded body.

6. Method according to claim 5, **characterised in that** the (ii) solvent or solvent mixture, each optionally comprising at least one monomer, comprises at least one short-chain alcohol having 1 to 4 C atoms, such as methanol, ethanol, ketone, such as acetone, an aqueous mixture of one afore-mentioned alcohol or ketone, optionally at least one monomer comprising a (meth-)acrylate having a (meth-)acrylate group or a mixture of the monomer with at least one alcohol or ketone.

7. Method according to claim 6, **characterised in that** the (ii) solvent or solvent mixture, each optionally comprising at least one monomer, comprises
a) at least one alcohol comprising methanol and/or ethanol,
b) at least one ketone comprising acetone,
c) at least one monomer comprising a (meth-)acrylate with a (meth-)acrylate group, optionally mixed with at least one alcohol and/or ketone, or
d) aqueous mixture of methanol or ethanol, or
e) aqueous mixture with acetone.

8. Method according to any one of the claims 5 to 7, **characterised in that** the mixture is transferred into a mould in (iii)., in particular the mould takes the shape of a cuboid, cube, rod, cylinder, strand, sphere, roundish convex shape, tetrahedron or polyhedron.

9. Moulded body obtainable according to a method according to any one of the claims 5 to 8.

10. Use of a moulded body according to any one of the claims 1 to 4 or 9 or obtained according to any one of the claims 5 to 8 for reproducible dosing of the polymeric particles.

11. Use of a moulded body according to any one of the claims 1 to 4 or 9 or obtained according to any one of the claims 5 to 8 together with at least one monomer comprising (meth-)acrylate with a (meth-)acrylate group comprising acrylate, methyl methacrylate, ethylene methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethylene acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate and/or a mixture containing at least one of said (meth-)acrylates for the production of a pasty castable or injectable polymeric dough being radiation- and/or thermally curable.

12. Use of a moulded body according to claims 1 to 4 or 9 for dosing the polymeric particles during the production of prostheses, dental prostheses, prosthetic materials, embedding compounds in histology, metallography, bone cements, prosthesis in veterinary medicine, of spacers, embedding compound for a porous substrate, for preparation of the microstructure of a substrate in metallography, for transparent section embedding in material testing, as embedding compound for testing printed circuit boards, as embedding compound for testing electronic components, as embedding compound for testing in semiconductor technology, as embedding compound for testing microelectronic components, as embedding compound for testing in optoelectronics, as embedding compound for testing in medical equipment technology and/or as embedding compound for testing medical instruments, as embedding compound or embedding material, in a material testing of a substrate.

13. Kit comprising at least one moulded body (a) and, separately, at least one defined amount of at least one pre-assembled polymerisable monomer (b),
(a) the at least one moulded body according to any one of the claims 1 to 4 or 9 or obtained according to any one of the claims 5 to 8
(a1) being formed from at least one organic polymer being soluble in (b), selected from acrylate and/or methacrylate polymer, PMMA poly(methyl-2-methylpropenoate), polymers based on monomers comprising at least one (meth-)acrylate group selected from methyl methacrylate, ethylene methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethylene acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate, a mixture containing at least one of said (meth-)acrylates and/or co-polymers containing at least one or at least two of the afore-mentioned monomers, and
(b) comprising at least one defined amount of a radically polymerisable monomer comprising a (meth-)acrylate group.

14. Kit according to claim 13, **characterised in that** (b) the at least one defined amount of a radically polymerisable monomer is selected from acrylate, methyl methacrylate, ethylene methacrylate, propyl methacrylate, butyl methacrylate, n-hexyl methacrylate, 2-phenoxyethyl methacrylate, isobornyl methacrylate, isodecyl methacrylate, polypropylene glycol monomethacrylate, tetrahydrofuryl methacrylate, methyl acrylate, ethylene acrylate, propyl acrylate, butyl acrylate, n-hexyl acrylate, 2-phenoxyethyl acrylate, isobornyl acrylate, isodecyl acrylate, polypropylene glycol monoacrylate, tetrahydrofuryl acrylate, hydroxyethyl acrylate, hydroxypropyl acrylate, hydroxyethyl methacrylate, hydroxypropyl methacrylate and/or a mixture containing at least one of said (meth-)acrylates, optionally the kit comprises
(c) photoinitiator and, if applicable, a content of activator, and optionally
(d) at least one photoinitiator and/or at least one hot- or auto-curing initiator.

## Revendications

1. Corps moulé pour le dosage reproductible des particules polymères, étant un corps moulé poreux tridimensionnel formé à partir des particules polymères, **caractérisé en ce que** les particules polymères comprennent des poudres, des grains, des pellets, des granulés, des particules sphériques, des extrudats et/ou des mélanges des différentes particules, et les particules sont fermement connectées l'une à l'autre, au moins en partie, aux points de contact des particules polymères, les particules étant collées, et les particules polymères consistant en polymères organiques, les polymères organiques étant sélectionnés parmi du polymère d'acrylate et/ou méthacrylate, du PMMA poly(2-méthylepropénoate de méthyle), des polymères basés sur des monomères comprenant au moins un group (méth-)acrylate sélectionné parmi du méthacrylate de méthyle, du méthacrylate d'éthylène, du méthacrylate de propyle, du méthacrylate de butyle, du méthacrylate de n-hexyle, du méthacrylate de 2-phenoxyéthyle, du méthacrylate d'isobornyle, du méthacrylate d'isodécyle, du monométhacrylate de polypropylène glycol, du méthacrylate de tetrahydrofuryle, de l'acrylate de méthyle, de l'acrylate d'éthylène, de l'acrylate de propyle, de l'acrylate de butyle, de l'acrylate de n-hexyle, de l'acrylate de 2-phenoxyéthyle, de l'acrylate d'isobornyle, de l'acrylate d'isodécyle, du monoacrylate de polypropylène glycol, de l'acrylate de tetrahydrofuryle, de l'acrylate d'hydroxyéthyle, de l'acrylate d'hydroxypropyle, du méthacrylate d'hydroxyéthyle, du méthacrylate d'hydroxypropyle, un mélange contenant au moins un desdites (méth-)acrylates et/ou des copolymères contenant au moins un ou au moins deux des monomères susmentionnés, et le corps moulé étant présent dans une forme tridimensionnelle géométriquement définie, et la porosité du corps moulé étant de sorte que le corps moulé absorbe, en particulière éponge, un liquide, dont le composant principal étant méthacrylate de méthyle, et le corps moulé se désintégrant, au moins en partie, en les particules polymères ou étant transformable en une pâte polymère coulable, pressable ou injectable après mélanger brièvement, obtenable par un procédé dans lequel les particules polymères sont traitées avec un solvant, un mélange de solvants, chacun facultativement conjointement avec un monomère de façon que seulement les surfaces des particules sont mouillées et les particules organiques sont solvatées ou mouillées superficiellement.

2. Corps moulé selon la revendication 1, **caractérisé en ce que** le corps moulé contient des monomères aux points de contact auxquelles les particules polymères sont connectées l'une à l'autre, au moins en partie, comprenant le (méth-)acrylate avec un group (méth)acrylate sélectionnée parmi de l'acrylate, du méthacrylate de méthyle, du méthacrylate d'éthylène, du méthacrylate de propyle, du méthacrylate de butyle, du méthacrylate de n-hexyle, du méthacrylate de 2-phenoxyéthyle, du méthacrylate d'isobornyle, du méthacrylate d'isodécyle, du monométhacrylate de polypropylène glycol, du méthacrylate de tetrahydrofuryle, de l'acrylate de méthyle, de l'acrylate d'éthylène, de l'acrylate de propyle, de l'acrylate de butyle, de l'acrylate de n-hexyle, de l'acrylate de 2-phenoxyéthyle, de l'acrylate d'isobornyle, de l'acrylate d'isodécyle, du monoacrylate de polypropylène glycol, de l'acrylate de tetrahydrofuryle, de l'acrylate d'hydroxyéthyle, de l'acrylate d'hydroxypropyle, du méthacrylate d'hydroxyéthyle, du méthacrylate d'hydroxypropyle et/ou un mélange contenant au moins un desdites (méth-)acrylates.

3. Corps moulé selon l'une des revendications 1 ou 2, **caractérisé en ce que** la surface spécifique du corps moulé s'élève à 80 % de la surface spécifique des particules polymères desquelles le corps moulé a été produit.

4. Corps moulé selon l'une des revendications 1 à 3, **caractérisé en ce que** le corps moulé prend substantiellement la forme d'un pavé droit, d'un cube, d'une barre, d'un cylindre, d'un écheveau, d'une sphère, d'une forme convexe arrondie, d'un tétraèdre ou d'un polyèdre.

5. Procédé pour la production du corps moulé pour le dosage reproductible des particules polymères selon l'une des revendications 1 à 4, dans lequel
(i). des particules polymères des polymères organiques, les particules polymères consistant en polymères organiques, les polymères organiques étant sélectionnés parmi du polymère d'acrylate et/ou méthacrylate, du PMMA poly(2-méthylepropénoate de méthyle), des polymères basés sur des monomères comprenant au moins un group (méth-)acrylate sélectionné parmi du méthacrylate de méthyle, du méthacrylate d'éthylène, du méthacrylate de propyle, du méthacrylate de butyle, du méthacrylate de n-hexyle, du méthacrylate de 2-phenoxyéthyle, du méthacrylate d'isobornyle, du méthacrylate d'isodécyle, du monométhacrylate de polypropylène glycol, du méthacrylate de tetrahydrofuryle, de l'acrylate de méthyle, de l'acrylate d'éthylène, de l'acrylate de propyle, de l'acrylate de butyle, de l'acrylate de n-hexyle, de l'acrylate de 2-phenoxyéthyle, de l'acrylate d'isobornyle, de l'acrylate d'isodécyle, du monoacrylate de polypropylène glycol, de l'acrylate de tetrahydrofuryle, de l'acrylate d'hydroxyéthyle, de l'acrylate d'hydroxypropyle, du méthacrylate d'hydroxyéthyle, du méthacrylate d'hydroxypropyle, un mélange contenant au moins un desdites (méth-)acrylates et/ou des copolymères contenant au moins un ou au moins deux des monomères susmentionnés, sont traités
(ii). avec un solvent ou un mélange de solvants, chacun comprenant facultativement au moins un monomère à obtenir un mélange,
(iii). mouler le mélange,
(iv). enlever le solvent ou le mélange de solvants,
(v). obtenir le corps moulé.

6. Procédé selon la revendication 5, **caractérisé en ce que** le (ii) solvent ou le mélange de solvants, chacun comprenant facultativement au moins un monomère, comprend au moins un alcool à chaîne courte ayant 1 à 4 atome C, tel que le méthanol, l'éthanol, une cétone, telle que l'acétone, un mélange aqueux d'un alcool susmentionné ou d'une cétone susmentionnée, facultativement au moins un monomère comprenant un (méth-)acrylate ayant un group (méth-)acrylate ou un mélange du monomère avec au moins un alcool ou une cétone.

7. Procédé selon la revendication 6, **caractérisé en ce que** le (ii) solvent ou le mélange de solvants, chacun comprenant facultativement au moins un monomère, comprend
a) au moins un alcool comprenant le méthanol et/ou l'éthanol,
b) au moins une cétone comprenant l'acétone,
c) au moins un monomère comprenant un (méth-)acrylate avec un group (méth-)acrylate, facultativement mélangé avec au moins un alcool et/ou une cétone, ou
d) un mélange aqueux du méthanol ou de l'éthanol, ou
e) un mélange aqueux avec l'acétone.

8. Procédé selon l'une des revendications 5 à 7, **caractérisé en ce que** le mélange est transféré dans un moule en (iii)., en particulière le moule prend la forme d'un pavé droit, d'un cube, d'une barre, d'un cylindre, d'un écheveau, d'une sphère, d'une forme convexe arrondie, d'un tétraèdre ou d'un polyèdre.

9. Corps moulé obtenable selon un procédé selon l'une des revendications 5 à 8.

10. Utilisation d'un corps moulé selon l'une des revendications 1 à 4 ou 9 ou obtenu selon l'une des revendications 5 à 8 pour le dosage reproductible des particules polymères.

11. Utilisation d'un corps moulé selon l'une des revendications 1 à 4 ou 9 ou obtenu selon l'une des revendications 5 à 8 conjointement avec au moins un monomère comprenant le (méth-)acrylate avec un group (méth-)acrylate comprenant l'acrylate, le méthacrylate de méthyle, le méthacrylate d'éthylène, le méthacrylate de propyle, le méthacrylate de butyle, le méthacrylate de n-hexyle, le méthacrylate de 2-phenoxyéthyle, le méthacrylate d'isobornyle, le méthacrylate d'isodécyle, le monométhacrylate de polypropylène glycol, le méthacrylate de tetrahydrofuryle, l'acrylate de méthyle, l'acrylate d'éthylène, l'acrylate de propyle, l'acrylate de butyle, l'acrylate de n-hexyle, l'acrylate de 2-phenoxyéthyle, l'acrylate d'isobornyle, l'acrylate d'isodécyle, le monoacrylate de polypropylène glycol, l'acrylate de tetrahydrofuryle, l'acrylate d'hydroxyéthyle, l'acrylate d'hydroxypropyle, le méthacrylate d'hydroxyéthyle, le méthacrylate d'hydroxypropyle et/ou un mélange contenant au moins un desdites (méth-)acrylates pour la production d'une pâte polymère pâteuse coulable ou injectable.

12. Utilisation d'un corps moulé selon les revendications 1 à 4 ou 9 pour le dosage des particules polymères pendant la production des prothèses, des prothèses dentaires, des matériaux prothétiques, des composés d'enrobage en histologie, métallographie, des ciments osseux, de la prothèse en médicine vétérinaire, des entretoises, du composé d'enrobage pour un substrat poreux, pour la préparation de la microstructure d'un substrat en métallographie, pour l'enrobage de section transparent en essaie des matériaux, en tant qu'un composé d'enrobage pour tester des cartes de circuit imprimé, en tant qu'un composé d'enrobage pour tester des composants électroniques, en tant qu'un composé d'enrobage pour tester en technologie des semi-conducteurs, en tant qu'un composé d'enrobage pour tester des composants microélectroniques, en tant qu'un composé d'enrobage en optoélectronique, en tant qu'un composé d'enrobage pour tester en technologie d'appareils médicale et/ou en tant qu'un composé d'enrobage pour tester des instruments médicaux, en tant qu'un composé d'enrobage ou qu'un matériau d'enrobage, en essaie des matériaux d'un substrat.

13. Kit comprenant un corps moulé (a) et, séparément, au moins une quantité définie d'au moins un monomère polymérisable préassemblé (b),
(a) l'au moins un corps moulé selon l'une des revendications 1 à 4 ou 9 ou obtenu selon l'une des revendications 5 à 8
(a1) étant formé à partir d'au moins un polymère organique étant soluble dans (b), sélectionné parmi du polymère d'acrylate et/ou méthacrylate, du PMMA poly(2-méthylepropénoate de méthyle), des polymères basés sur des monomères comprenant au moins un group (méth-)acrylate sélectionné parmi du méthacrylate de méthyle, du méthacrylate d'éthylène, du méthacrylate de propyle, du méthacrylate de butyle, du méthacrylate de n-hexyle, du méthacrylate de 2-phenoxyéthyle, du méthacrylate d'isobornyle, du méthacrylate d'isodécyle, du monométhacrylate de polypropylène glycol, du méthacrylate de tetrahydrofuryle, de l'acrylate de méthyle, de l'acrylate d'éthylène, de l'acrylate de propyle, de l'acrylate de butyle, de l'acrylate de n-hexyle, de l'acrylate de 2-phenoxyéthyle, de l'acrylate d'isobornyle, de l'acrylate d'isodécyle, du monoacrylate de polypropylène glycol, de l'acrylate de tetrahydrofuryle, de l'acrylate d'hydroxyéthyle, de l'acrylate d'hydroxypropyle, du méthacrylate d'hydroxyéthyle, du méthacrylate d'hydroxypropyle, un mélange contenant au moins un desdites (méth-)acrylates et/ou des copolymères contenant au moins un ou au moins deux des monomères susmentionnés, et
(b) comprenant au moins une quantité définie d'un polymère polymérisable par voie radicalaire comprenant un group (méth-)acrylate.

14. Kit selon la revendication 13, **caractérisé en ce que** (b) l'au moins une quantité définie d'un monomère polymérisable à voie radicalaire est sélectionnée parmi de l'acrylate, du méthacrylate de méthyle, du méthacrylate d'éthylène, du méthacrylate de propyle, du méthacrylate de butyle, du méthacrylate de n-hexyle, du méthacrylate de 2-phenoxyéthyle, du méthacrylate d'isobornyle, du méthacrylate d'isodécyle, du monométhacrylate de polypropylène glycol, du méthacrylate de tetrahydrofuryle, de l'acrylate de méthyle, de l'acrylate d'éthylène, de l'acrylate de propyle, de l'acrylate de butyle, de l'acrylate de n-hexyle, de l'acrylate de 2-phenoxyéthyle, de l'acrylate d'isobornyle, de l'acrylate d'isodécyle, du monoacrylate de polypropylène glycol, de l'acrylate de tetrahydrofuryle, de l'acrylate d'hydroxyéthyle, de l'acrylate d'hydroxypropyle, du méthacrylate d'hydroxyéthyle, du méthacrylate d'hydroxypropyle et/ou un mélange contenant au moins un desdites (méth-)acrylates, facultativement le kit comprend
(c) du photoinitiateur et, le cas échéant, une teneur de l'activateur, et facultativement
(d) au moins un photoinitiateur et/ou au moins un initiateur durcissant à chaud ou auto-durcissant.
